# EUROPÄISCHE PATENTSCHRIFT

(11) **EP 2 877 100 B1**
(45) Veröffentlichungstag und Bekanntmachung des Hinweises auf die Patenterteilung: **20.01.2021**
(21) Anmeldenummer: 13744480.8
(22) Anmeldetag: 24.07.2013
(51) Int. Cl.: A61B 34/00, A61B 17/00

(54) **SCHAFT FÜR MEDIZINISCHES INSTRUMENT MIT BEWEGLICHEN ABSCHNITTE**
SHAFT FOR MEDICAL INSTRUMENTS, COMPRISING MOVABLE SECTIONS
TIGE POUR INSTRUMENT MÉDICAL COMPRENANT DES SECTIONS MOBILES

(30) Priorität: 24.07.2012 DE 102012212997
(43) Veröffentlichungstag der Anmeldung: 03.06.2015
(73) Patentinhaber: Richard Wolf GmbH, 75438 Knittlingen (DE)
(72) Erfinder: TEICHTMANN, Elmar, 75015 Bretten (DE); WEHRHEIM, Frank, 75015 Bretten (DE)
(74) Vertreter: Patentanwälte Vollmann Hemmer Lindfeld Partnerschaft mbB
(86) Internationale Anmeldenummer: PCT/EP2013/065616
(87) Internationale Veröffentlichungsnummer: WO 2014/016337

(56) Entgegenhaltungen:
- EP-A1- 0 612 496
- EP-A1- 2 371 299
- WO-A1-2012/040981
- WO-A2-2009/145572
- KR-A- 20100 131 338
- US-A1- 2005 256 533
- US-A1- 2006 199 999
- US-A1- 2011 152 879
- US-A1- 2012 123 200
- US-B1- 6 309 403

## Beschreibung

Die Erfindung betrifft ein medizinisches Instrument mit den im Oberbegriff des Anspruchs 1 angegebenen Merkmalen.

Es sind medizinische Instrumente, insbesondere endoskopische Instrumente bekannt, welche einen Schaft aufweisen, welcher in zumindest einem Bereich abwinkelbar ausgebildet ist. Dabei sind Schäfte bekannt, welche einzelne Schwenkgelenke aufweisen oder aber bewegliche Schäfte, welche eine Vielzahl von gegeneinander beweglichen Schaftabschnitten aufweisen.

In jüngerer Zeit gibt es Bestrebungen, Robotersysteme für Operationen einzusetzen. Bei diesen ist es zwingend erforderlich, dass die Steuerung die genaue Instrumentenposition kennt. Bei Schäften, welche mehrere gegeneinander abwinkelbare Schaftabschnitte aufweisen, welche über Zugseile verschwenkt werden, besteht jedoch das Problem, dass die einzelnen Schaftabschnitte keine genau definierte Bewegung zueinander ausführen. Vielmehr hängt die Bewegung davon ab, wie die Abschnitte beispielsweise an angrenzendem Gewebe anliegen. Ein solches System ist für einen Roboter basiertes Operationssystem daher nicht geeignet.

Die EP 0 612 496 A1 beschreibt einen Schaft für medizinische Instrumente, welcher aus einer Mehrzahl aneinandergereihter Schaftabschnitte zusammengesetzt ist. Dabei kämmen die Schaftabschnitte über bogenförmige Verzahnungen miteinander. Die Verzahnungen sind so ausgebildet, dass stets die Verzahnung eines ersten Schaftabschnittes konvex ausgebildet ist und in eine konkave Verzahnung an einem gegenüberliegenden Schaftabschnitt eingreift. Eine solche Anordnung ermöglicht nur eine geringe Biegbarkeit.

Die WO 2009/145572 A2 beschreibt ein Instrument für die minimalinvasive Chirurgie, welches an seinem distalen Ende und an seinem proximalen Ende jeweils ein in zwei Richtungen schwenkbares Gelenk aufweist. Die Gelenke sind dabei aus beweglichen Abschnitten ausgebildet, welche miteinander kämmende Zahnradabschnitte aufweisen, wobei die gelenkigen Abschnitte über starre Verbindungsstege miteinander verbunden sind, welche jeweils um Gelenkachsen drehbar sind, welche sich konzentrisch zu den Zahnradabschnitten erstrecken. Dieser Aufbau ist aufwendig zu montieren.

Ein ähnlicher Aufbau ist aus KR 2010-0131338 A bekannt. Bei den dort gezeigten Gelenken sind die aufeinander abrollenden Gelenkelemente durch umschlingende Bänder miteinander verbunden.

Im Hinblick auf diese Problematik ist es Aufgabe der Erfindung, ein medizinisches Instrument mit einem Schaft, welcher zumindest zwei benachbarte gegeneinander bewegliche Schaftabschnitte aufweist, dahingehend zu verbessern, dass die Schaftabschnitte definiert zueinander verschwenken.

Diese Aufgabe wird durch ein medizinisches Instrument mit den in Anspruch 1 angegebenen Merkmalen gelöst. Bevorzugte Ausführungsformen ergeben sich aus den Unteransprüchen, der nachfolgenden Beschreibung sowie den beigefügten Figuren.

Das erfindungsgemäße medizinische Instrument weist in bekannter Weise zumindest einen vorzugsweise hohlen Schaft auf, welcher zumindest zwei benachbarte gegeneinander bewegliche Schaftabschnitte aufweist. D. h. die beiden Schaftabschnitte können gegeneinander verschwenken. Erfindungsgemäß sind die zumindest zwei beweglichen Schaftabschnitte so ausgebildet, dass sie entweder über Verzahnungen formschlüssig kämmend miteinander in Eingriff sind oder reibschlüssig miteinander in Eingriff sind, sodass sie schlupffrei aufeinander abrollen. D. h. bei der Schwenkbewegung rollen die beiden beweglichen Schaftabschnitte nach Art eines Zahnradgetriebes oder reibschlüssig, schlupffrei aufeinander ab und sind stets definiert formschlüssig oder kraftschlüssig zueinander in Eingriff. D. h. die beiden Schaftabschnitte führen stets eine definierte, durch die Verzahnung oder den Reibschluss vorgegebene Bewegung relativ zueinander aus, wenn sie beispielsweise durch Zugelemente zu einer Auslenkung veranlasst werden.

Der Schaft weist eine Mehrzahl gegeneinander beweglicher Schaftabschnitte auf, von denen jeweils zwei zueinander benachbarte Schaftabschnitte über Verzahnungen oder reibschlüssig aneinander abrollend, kämmend miteinander in Eingriff sind. So werden mehrere bzw. viele Gelenkstellen zwischen den einzelnen aneinander angrenzenden bzw. zueinander benachbarten Schaftabschnitten geschaffen, wobei in diesen Gelenkstellen aufgrund des kraft- oder formschlüssigen Eingriffs stets eine definierte Abrollbewegung der einander angrenzenden Schaftabschnitte zueinander erzielt wird. Eine solche definierte Bewegung macht ein solches flexibles Instrument auch für die Roboter basierte Chirurgie verwendbar, da sich ein solcher Schaft so steuern bzw. bewegen lässt, dass die Bewegungen stets so definiert sind, dass der Steuerung die Positionierung des Schaftes und insbesondere des distalen Endes des Schaftes bekannt ist.

Die Verzahnungen sind so ausgebildet, dass zwei benachbarte Schaftabschnitte an ihren einander gegenüberliegenden Stirnenden mit korrespondierenden Verzahnungen versehen sind, welche so miteinander in Eingriff sind, dass die beiden Schaftabschnitte gegeneinander verschwenken können. Diese Schwenkbewegung kann insbesondere durch ein Abrollen der Schaftabschnitte aufeinander bzw. in den Verzahnungen, welche miteinander in Eingriff sind, erfolgen.

Im Falle, dass ein reibschlüssiger Eingriff der Schaftabschnitte vorgesehen ist, weisen die Schaftabschnitte jeweils Reib- bzw. Eingriffsflächen auf, welche reibschlüssig aneinander anliegen, d. h. an zwei benachbarten Schaftabschnitten sind an ihren einander gegenüberliegenden Stirnenden zueinander korrespondierende Anlageflächen bzw. Reibflächen ausgebildet, welche reibschlüssig so miteinander in Eingriff sind, dass die beiden Schaftabschnitte gegeneinander verschwenken können. Dabei rollen die Reibflächen schlupffrei aufeinander ab. Die zueinander korrespondierenden und aneinander anliegenden Reibflächen können aufgeraute oder elastische Oberflächenstrukturen aufweisen, welche einen reibschlüssigen Eingriff und ein schlupffreies Abrollen sicherstellen.

Um dies zu ermöglichen, sind zumindest einzelne Verzahnungen oder Reibflächen bogenförmig ausgebildet. D. h. zumindest eine der beiden Verzahnungen oder Reibflächen, welche miteinander in Eingriff sind, ist bogenförmig ausgebildet. Bevorzugt sind beide miteinander kämmenden Verzahnungen bzw. im Eingriff befindlichen Reibflächen bogenförmig ausgebildet, sodass eine Abrollbewegung aufeinander erfolgt.

Dabei weisen die miteinander kämmenden Verzahnungen bzw. aneinander abrollenden Reibflächen an den aneinander gegenüberliegenden Stirnenden zweier benachbarter Schaftabschnitte weiter bevorzugt denselben Krümmungsradius auf. So wird eine gleichmäßige Abwinkelung erreicht.

Ferner sind die miteinander kämmenden Verzahnungen bzw. die aneinander abrollenden Reibflächen an den einander gegenüberliegenden Stirnenden zweier benachbarter Schaftabschnitte in entgegengesetzten Richtungen gekrümmt. D. h. es gibt zwei bogenförmige Außenverzahnungen, welche jeweils den Abschnitt eines Zahnrades bilden, oder bogenförmige Radabschnitte. Die Bogenform entspricht dabei, wie oben beschrieben, vorzugsweise einer Kreisbogenform. So wird ein Eingriff der zwei zueinander beabstandeten Schaftabschnitte nach Art eines Zahnradgetriebes oder ein reibschlüssiges Abrollen nach Art eines Reibradgetriebes erreicht.

Die benachbarten Schaftabschnitte weisen an ihren einander zugewandten Stirnenden jeweils an zwei diametral entgegengesetzten Seiten eine Verzahnung bzw. Reibfläche auf. Die Verzahnungen bzw. Reibflächen erstrecken sich somit bevorzugt in zumindest zwei zueinander und zur Längsachse des Schaftabschnittes parallelen Ebenen, welche die Schwenkebenen bilden, in denen die zwei zueinander parallelen Schaftabschnitte zueinander verschwenken können. Die Verzahnungen bzw. Reibflächen erstrecken sich dabei bevorzugt in Ebenen, welche tangential oder sehnenförmig zu der Außenwandung des Schaftabschnittes angeordnet sind.

Insgesamt erstrecken sich die Verzahnungen bzw. Reibflächen somit vorzugsweise an den zwei diametral entgegengesetzten Seiten bogenförmig um dieselbe Achse. Dabei weisen sie wie beschrieben, eine Kreisbogenform auf. An der gegenüberliegenden Seite eines benachbarten Schaftabschnittes sind korrespondierend bevorzugt eine identisch ausgebildete Verzahnung bzw. Reibflächen gelegen.

Besonders bevorzugt weist der Schaft zumindest drei gegeneinander bewegliche Schaftabschnitte auf, von denen die jeweils benachbarten Schaftabschnitte über Verzahnungen kämmend oder reibschlüssig aneinander abrollend miteinander in Eingriff sind. Dabei können die Verzahnungen oder Reibflächen in der vorangehend beschriebenen Weise ausgebildet sein. Bei dieser bevorzugten Ausführungsform sind weiter bevorzugt der erste und der zweite Schaftabschnitt in einer ersten Schwenkebene und der zweite und der dritte Schaftabschnitt in einer zweiten Schwenkebene zueinander beweglich. D. h. die Schwenkebenen zwischen den einzelnen Schaftabschnitten wechseln einander ab, sodass eine Beweglichkeit in verschiedenen Richtungen zueinander erreicht wird. Bevorzugt erstrecken sich die erste und die zweite Schwenkebene normal zueinander, sodass sich die einzelnen Schwenkebenen jeweils um 90° bezüglich der Längsachse des Schaftes versetzt zueinander abwechseln. Dadurch wird eine Auslenkung des Schaftes in allen Raumrichtungen möglich, in dem die Schwenkbewegungen in den zwei Richtungen kombiniert bzw. überlagert werden. Um diese abwechselnde Ausrichtung der Schwenkebenen zwischen den einzelnen Schaftabschnitten zu erreichen, sind vorzugsweise die Erstreckungsebenen, in welchen die bogenförmigen Verzahnungen bzw. Reibflächen gelegen sind, von einer Schnittstelle zur nächsten jeweils um 90° versetzt zueinander angeordnet. D. h. zwischen dem ersten und dem zweiten Schaftabschnitt sind die bogenförmigen Verzahnungen bzw. Reibflächen in Ebenen parallel zu der ersten Schwenkebene angeordnet, während die bogenförmigen Verzahnungen bzw. Reibflächen zwischen dem zweiten und dritten Schaftabschnitt parallel zu der zweiten Schwenkebene angeordnet sind. Bei mehr als drei Schaftabschnitten setzt sich dies dann entsprechend fort.

Die Verzahnungen bzw. Reibflächen sind dabei bogenförmig ausgebildet und die Verzahnungen bzw. Reibflächen zwischen dem ersten und dem zweiten Abschnitt sind bevorzugt um Achsen, d. h. besonders bevorzugt um dieselbe Achse gekrümmt, welche sich normal zu den Achsen bzw. der Achse erstrecken, um welche die Verzahnungen bzw. Reibflächen zwischen dem zweiten und dem dritten Schaftabschnitt gekrümmt sind. Diese Achsen, um welche die Verzahnungen bzw. Reibflächen gekrümmt sind, erstrecken sich normal zu den jeweiligen Schwenkebenen, in welchen die Schwenkbewegungen zwischen den zwei benachbarten Schaftabschnitten ausgeführt werden.

Gemäß einer besonders bevorzugten Ausführungsform sind die zumindest zwei beweglichen Schaftabschnitte über Verzahnungen kämmend miteinander in Eingriff und rollen gleichzeitig über bogenförmige Anlageflächen aneinander ab. Das heißt, an den zwei Schaftabschnitten sind an einander zugewandten Seiten jeweils zumindest eine bogenförmige Verzahnung und zumindest eine bogenförmige Anlagefläche ausgebildet. Dabei greifen die Verzahnungen ineinander ein und die bogenförmigen Anlageflächen, welche einander zugewandt sind, liegen aneinander an. Diese Ausgestaltung hat den Vorteil, dass über die bogenförmigen Anlageflächen die Verzahnungen beim Verschwenken in einem definierten Abstand zueinander gehalten werden, das heißt, es wird ein konstanter Achsabstand zwischen den Schwenkachsen der beiden Schaftabschnitte eingehalten. Dieser wird durch die bogenförmigen Anlageflächen definiert.

Für den Fall, dass mehr als zwei Abschnitte vorgesehen sind, ist weiter bevorzugt eine derartige Ausgestaltung bestehend aus bogenförmigen Verzahnungen und bogenförmigen Anlageflächen, welche für einen konstanten Achsabstand sorgen, jeweils zwischen den aneinander angrenzenden Schaftabschnitten ausgebildet. Dabei können die Verzahnungen in der vorangehend beschriebenen Weise ausgestaltet und angeordnet sein.

Weiter bevorzugt ist gleichzeitig eine seitliche Führung vorgesehen, welche ein Verschieben aneinander angrenzender Schaftabschnitte in Richtung der Schwenkachse verhindert. Eine solche seitliche Führung kann durch eine an die bogenförmige Anlagefläche angrenzende, das heißt sich quer zu der Anlagefläche erstreckende Wandung bzw. Flanke gebildet werden. Beispielsweise können die Köpfe der Zähne in radialer Richtung über eine angrenzende Anlagefläche vorstehen, sodass zwischen den Zähnen Seitenflanken entstehen, an welchen die überstehenden Zähne eines gegenüberliegenden Schaftabschnittes bei seitlicher Bewegung anstoßen, sodass diese begrenzt oder verhindert wird.

Weiter bevorzugt weist jeder Schaftabschnitt zumindest eine bogenförmige Anlagefläche und eine Verzahnung auf, wobei die bogenförmige Anlagefläche zweckmäßigerweise parallel zu der Verzahnung gekrümmt ist. Dadurch wird erreicht, dass in jeder Winkelposition beim Verschwenken der Achsabstand zwischen den Schaftabschnitten konstant gehalten wird. Besonders bevorzugt hat die bogenförmige Anlagefläche denselben Radius bzw. Krümmungsradius wie der Teilkreis der Verzahnung.

Um die einzelnen Schaftabschnitte mit ihren Verzahnungen oder Reibflächen in Anlage bzw. in Eingriff zu halten, sind Spannelemente vorgesehen, welche sich in Axialrichtung des Schaftes erstrecken. Dies sind Spannseile, welche über Federelemente so vorgespannt sind, dass die Schaftabschnitte auch bei Schwenkbewegungen stets in Anlage gehalten werden, sodass Verzahnungen kämmend in Eingriff bleiben bzw. Reibflächen reibschlüssig in Anlage gehalten werden, sodass ein Abrollen ohne Schlupf stattfinden kann. Auch können die Schaftabschnitte über die nachfolgend beschriebenen Zugelemente in Anlage gehalten werden.

Die Auslenkung der einzelnen Schaftabschnitte erfolgt Zugdrähte bzw. Zugseile. Dazu sind zumindest zwei sich in Längsrichtung des Schaftes erstreckende Zugdrähte vorgesehen, welche mit ihrem distalen Ende an zwei diametral entgegengesetzten Seiten eines distalen Schaftabschnittes befestigt sind. Diese beiden diametral entgegengesetzten Seiten, an welchen die Zugdrähte befestigt und gelegen sind, befinden sich dabei bevorzugt in der Schwenkebene, innerhalb welcher die jeweiligen Schaftabschnitte zueinander verschwenken sollen. Durch Zug an einem der Zugdrähte erfolgt dann eine Auslenkung des Schaftes in die radiale Richtung, an welcher der sich verkürzende Zugdraht gelegen ist. Bei Zug an dem anderen Zugdraht erfolgt eine Auslenkung in die entgegengesetzte Richtung. Der jeweils zweite Zugdraht wird entsprechend gelockert bzw. gelenkt. Diese Auslenkung über Zugdrähte entspricht im Wesentlichen der von flexiblen Endoskopen her bekannten Auslenkung.

Um eine Auslenkung in zwei unterschiedlichen, insbesondere um 90° zueinander versetzten Raumrichtungen zu ermöglichen, ist es weiter bevorzugt, zumindest vier Zugdrähte vorzusehen, welche jeweils um 90° bezüglich der Längsachse versetzt an einem distalen Schaftabschnitt befestigt sind. Dabei liegen zwei der Zugdrähte diametral entgegengesetzt bevorzugt in der ersten beschrieben Schwenkebene und die zwei anderen Zugdrähte diametral entgegengesetzt in einer um 90° um die Längsachse des Schaftes versetzten Schwenkebene. D. h. zwei der Zugdrähte sind für die Schwenkbewegung in der ersten Schwenkebene und zwei der Zugdrähte für die Schwenkbewegung in der zweiten Schwenkebene zuständig. Wenn im Schaft mehrere gegeneinander bewegliche Schaftabschnitte angeordnet sind, welche abwechselnd in den zwei unterschiedlichen Schwenkebenen zueinander beweglich sind, wird bei Betätigung des ersten Paares von Zugdrähten eine Schwenkbewegung jeweils nur in jedem zweiten Schwenkgelenk erfolgen, welches eine Schwenkbewegung in dieser Schwenkebene zulässt. Bei Betätigung des anderen Paares von Zugdrähten erfolgt dann eine Schwenkbewegung in der normal gerichteten Schwenkebene in den jeweils anderen Schwenkgelenken.

Vorzugsweise sind vier Zugdrähte vorgesehen, welche an ihren distalen Enden miteinander verbunden sind. Die Verbindung der Zugdrähte erleichtert die Montage. Insbesondere sind alle Zugdrähte bzw. Steuerseile in einem distalen Schaftabschnitt, d. h. dem letzten beweglichen Schaftabschnitt am distalen Ende miteinander verbunden. So können die Zugdrähte ausgehend vom distalen Ende einfach durch die vorgesehenen Führungen und Ausnehmungen der sich proximalseitig anschließenden Schaftabschnitte bis zum proximalen Ende gefädelt werden. Bevorzugt können die vier Zugdrähte dabei als zwei U-förmige Schlaufen ausgebildet sein, welche sich kreuzen und in ihrem Kreuzungs- bzw. Mittelpunkt miteinander verbunden sind. Diese Verbindung kann beispielsweise durch Verschweißen oder Verkleben oder Verklemmen erfolgen.

Wenn die Zugdrähte nicht am distalen Schaftabschnitt angelenkt werden, sondern an weiter proximalwärts gelegenen Schaftabschnitten, so wird es auch möglich, über entsprechende Paare von Zugdrähten definiert einzelne Schaftabschnitte gegeneinander zu bewegen. D. h. für jedes Gelenk bzw. jeden bewegbaren Schaftabschnitt wird ein Paar von Zugdrähten vorgesehen, welches in der Schwenkebene dieses Schaftabschnittes an diametral entgegengesetzten Seiten des Schaftabschnittes befestigt ist. So kann eine definierte schlangenförmige Auslenkung des Instrumentes erreicht werden.

In denjenigen Schaftabschnitten, welche proximalseitig des distalen Schaftabschnittes bzw. desjenigen Schaftabschnittes gelegen sind, an welchem die jeweiligen Zugdrähte befestigt sind, sind weiter bevorzugt Führungen für diese Zugdrähte ausgebildet. So wird erreicht, dass die Zugdrähte definiert in der Schaftwandung bzw. an der Schaftwandung geführt werden. Darüber hinaus bieten die Führungen Anlagepunkte, welche das Auslenken in definierter Weise durch Zug an den Zugdrähten erst ermöglichen. So werden Kraftangriffspunkte für die Zugdrähte geschaffen.

Gemäß einer weiteren bevorzugten Ausführungsform weisen die zumindest zwei benachbarten zueinander beweglichen Schaftabschnitte eine Seitenführung auf. D. h. die zwei Schaftabschnitte sind über eine sich parallel zur Schwenkebene erstreckende Seitenführung miteinander in Eingriff. Die Seitenführung kann von einer oder mehreren Führungsflächen gebildet werden. Vorzugsweise steht zumindest ein Steg von einem der Schaftabschnitte vor und greift in eine gegenüberliegende Nut ein. Dabei erstrecken sich der Steg und die Nut in der Schwenkebene bzw. parallel zu der Schwenkebene. Gemäß einer ersten bevorzugten Ausführungsform ist lediglich eine Seitenführung in Form eines Steges vorgesehen, welcher vorzugsweise im Mittelbereich gelegen ist. Gemäß einer alternativen Ausführungsform können auch zwei in Durchmesserrichtung voneinander beabstandete Seitenführungen, welche sich parallel zueinander erstrecken, vorgesehen sein.

Wie oben ausgeführt, ist der Schaft des medizinischen Instrumentes gemäß einer bevorzugten Ausführungsform hohl ausgebildet. Dazu können die zumindest zwei beweglichen Schaftabschnitte jeweils einen sich in axialer Richtung durch den Schaftabschnitt hindurch erstreckenden zentralen Hohlraum aufweisen, wobei die Hohlräume der mehreren Schaftabschnitte miteinander fluchten. Auf diese Weise wird durch die beweglichen Schaftabschnitte hindurch, ein durchgehender, sich in axialer Richtung erstreckender Hohlraum geschaffen. Dieser Hohlraum bzw. Kanal erlaubt die Führung und optionale Befestigung weiterer Baugruppen, wie beispielsweise Lichtwellenleiter, Bildleiter, elektrische Leitungen, Schläuche oder Rohre zum Führen von Medien, wie Gas oder Flüssigkeiten, Schläuche oder Rohre zum Führen von Anwendungswerkzeugen wie Zangen, Körbe, Laserfasern, Kraftübertragungselemente wie Stangen, Züge, Mikroantriebe wie Piezoantriebe, auf Formgedächtnis basierende Aktoren, elektroaktive Polymeraktoren, dieleketrische Elastomeraktoren, magnetostriktive Antriebe, magnetorheologische Antriebe, Pneumatik- und Hydraulikantriebe, sowie elektromagnetische Antriebe usw.

Nachfolgend wird die Erfindung beispielhaft anhand der beigefügten Figuren beschrieben. In diesen zeigt:
- Fig. 1: eine schematische perspektivische Gesamtansicht des distalen Endes eines medizinisches Instrumentes,
- Fig. 2: eine Ansicht gemäß Fig. 1 ohne umhüllenden Schutzschlauch,
- Fig. 3: schematisch den Schaftaufbau des Instrumentes gemäß Fig. 1 und 2,
- Fig. 4: schematisch die Abwinklung des Schaftes gemäß Fig. 1 - 3,
- Fig. 5: eine schematische Seitenansicht einer zweiten Ausführungsform der Erfindung,
- Fig. 6: die Abwinklung des Schaftes gemäß Fig. 5,
- Fig. 7: schematisch den Aufbau eines Schaftes gemäß einer dritten Ausführungsform der Erfindung,
- Fig. 8: einen Schaftabschnitt gemäß einer weiteren Ausführungsform der Erfindung,
- Fig. 9: einen Schaftabschnitt einer weiteren Ausführungsform der Erfindung,
- Fig. 10: schematisch die Abwinklung zweier beweglicher Schaftabschnitte,
- Fig. 11: schematisch in einer perspektivischen Ansicht einen seriell abwinkelbaren Schaft gemäß der Erfindung,
- Fig. 12: eine Seitenansicht des Schaftes gemäß Fig. 11,
- Fig. 13: schematisch einen in zwei Richtungen abwinkelbaren Schaft,
- Fig. 14: schematisch den Aufbau eines Schaftes gemäß einer weiteren Ausführungsform der Erfindung,
- Fig. 15: eine Detailansicht des Schaftes gemäß Fig. 14 ohne den distalen Schaftabschnitt,
- Fig. 16A - 16B: in zwei entgegengesetzten Draufsichten und einer Seitenansicht den zentralen beweglichen Schaftabschnitt der Ausführungsform gemäß Fig. 14 und 15,
- Fig. 17: schematisch einen Endbereich eines Schaftabschnittes gemäß einer weiteren Ausführungsform der Erfindung,
- Fig. 18: eine Vergrößerung des Ausschnittes XVIII in Fig. 17,
- Fig. 19: schematisch in einer perspektivischen Ansicht die Anlage zweier Schaftabschnitte gemäß Fig. 17 und 18,
- Fig. 20: eine Schnittansicht der Anordnung in Fig. 19,
- Fig. 21: schematisch einen Endbereich eines Schaftabschnittes gemäß einer weiteren Ausführungsform der Erfindung,
- Fig. 22: vergrößert den Ausschnitt XXII in Fig. 21,
- Fig. 23: schematisch in einer perspektivischen Ansicht die Anlage zweier Schaftabschnitte gemäß Fig. 21 und 22,
- Fig. 24: eine Draufsicht auf die Anordnung gemäß Fig. 23, und
- Fig. 25: eine Schnittansicht entlang der Linie XXV-XXV in Fig. 24.

Das erfindungsgemäße medizinische Instrument ist vorzugsweise ein endoskopisches Instrument und weist in bekannter Weise einen Schaft 2 auf, dessen distaler Schaftbereich 4 anwinkelbar ausgebildet ist. Am distalen Ende des Schaftes ist ein Zangenmaul 6 angeordnet. Das Zangenmaul 6 ist in bekannter Weise vom proximalen Ende des Schaftes 2 (das proximale Ende ist hier nicht gezeigt) über eine Handhabe oder elektrische Antriebe bewegbar. Dazu können im Inneren des Schaftes 2 Betätigungselemente, wie Zug- oder Druckelemente in axialer Richtung X verlaufen.

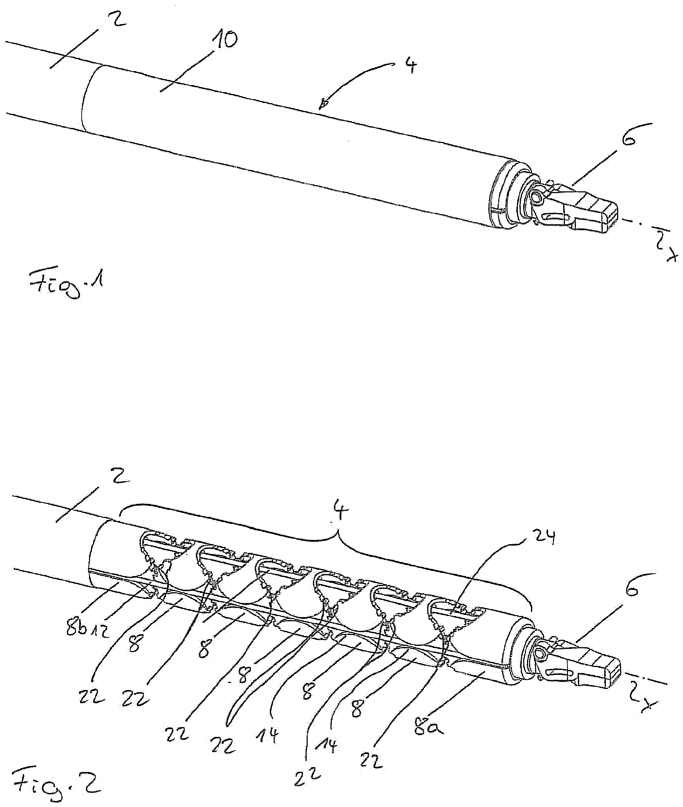

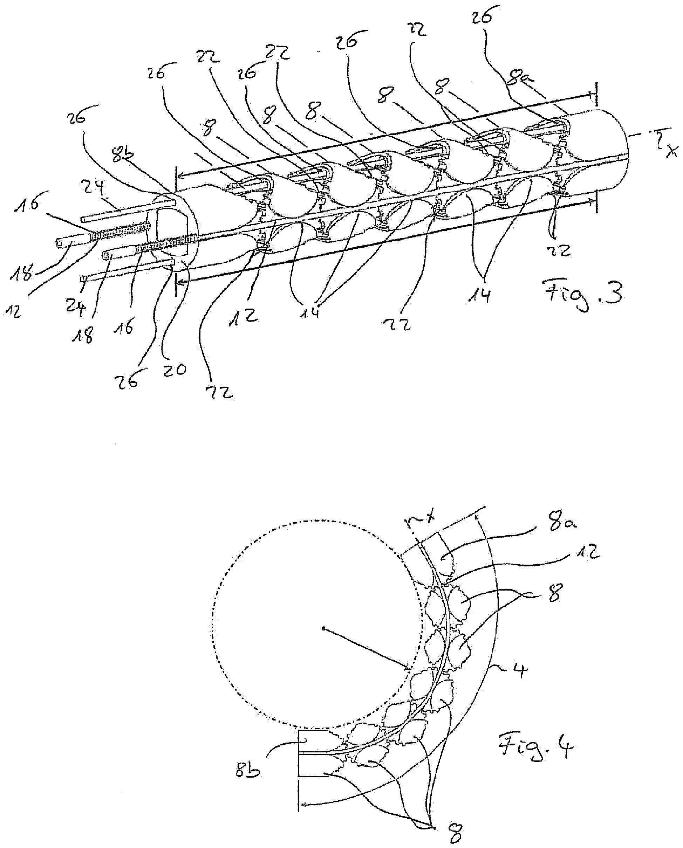

Der distale Schaftbereich 4 besteht aus mehreren axial aneinander angrenzend angeordneten Schaftabschnitten 8. Die Schaftabschnitte 8 sind an ihrem Außenumfang, wie in Fig. 1 schematisch gezeigt, von einem Schutzschlauch umgeben. Die Schaftabschnitte 8 werden in axialer Richtung über zwei diametral beabstandete Spannseile 12 aneinander in Anlage gehalten. Dazu erstrecken sich die Spannseile 12, wie in Fig. 2 und 3 gezeigt, durch Führungsnuten 14 in den Wandungen der Schaftabschnitte 8. An dem distalen Schaftabschnitt 8a, welcher am nächsten zum Zangenmaul 6 gelegen ist, sind die Spannseile 12 fixiert. Am entgegengesetzten Axialende sind auf den Spannstäben bzw. Spannseilen 12 Druckfedern 16 angeordnet, welche sich mit einem Axialende an einer Hülse 18, welche auf dem Spannseil 12 fixiert ist, abstützen und mit dem entgegengesetzten Axialende an der proximalen Endfläche 20 des proximalen Schaftabschnittes 8b des distalen Schaftbereiches 4 abstützen. Die Druckfedern 16 bewirken, dass die Schaftabschnitte 8 auf den Spannstäben bzw. Spannseilen 12 gegeneinander in Anlage gedrückt werden.

Die einzelnen Schaftabschnitte 8 sind jeweils über Verzahnungen 22 miteinander in Eingriff. Die Verzahnungen 22 sind an den einzelnen Schaftelementen 8 an zwei diametral entgegengesetzten Seiten ausgebildet und zwar an den Seiten, an welchen auch die Führungsnuten 14 gelegen sind, durch welche sich die Spannseile 12 erstrecken. Die Verzahnungen 22 sind jeweils bogenförmig ausgebildet, sodass sie den Abschnitt eines Zahnrades bilden. Die Achsen, um die die Verzahnungen 22 bogenförmig gekrümmt sind, erstrecken sich normal zu der Längsachse X des Schaftes 2 in diametraler Richtung durch die Schaftabschnitte 8 hindurch und kreuzen somit die Spannseile 12. Die Verzahnungen 22 erlauben es, dass die Schaftabschnitte 8, welche zueinander benachbart sind, bzw. aneinander angrenzen, über die Verzahnungen 22 aufeinander abrollen können. So kann der Schaftbereich 4, wie in Fig. 4 gezeigt ist, gekrümmt werden.

Die Krümmung wird durch zwei Zugzeile 24 erreicht, welche sich durch Führungen in Form von Löchern 26 in den Wandungen der Schaftabschnitte 8 in axialer Richtung erstrecken. Die beiden Zugsseile 24 sind an zwei diametral entgegensetzten Seiten der Schaftabschnitte 8 um 90° versetzt zu den Seiten, an welchen die Spannseile 12 angeordnet sind, angeordnet. Eine diametrale Verbindungslinie zwischen den Zugseilen 24 erstreckt sich somit normal zu den Achsen, um welche die Verzahnungen 22 gekrümmt sind. Die Zugseile 24 sind ebenfalls am distalseitigen Schaftabschnitt 8a, welcher am nächsten zu dem Zangenmaul 6 und somit dem distalen Ende des Schaftes 2 gelegen ist, fest verbunden. Sie erstrecken sich durch den Schaft 2 bis zum proximalen Ende, welches hier nicht gezeigt ist und sind dort in bekannter Weise mit Antriebselementen oder Betätigungselementen verbunden. Je nachdem, welches der Zugseile 24 in proximaler Richtung gezogen und somit in dem Schaftbereich 4 verkürzt wird, kann der Schaftbereich 4 zu einer Richtung hin ausgelenkt werden. Das diametral entgegengesetzt gelegene Zugseil 24 wird entsprechend gelängt. Eine Auslenkung in die andere Richtung erfolgt entsprechend durch Kürzung des anderen Zugseils 24. Die Zugseile 24 könnten auch als Zug-Druckelemente ausgebildet sein. Dann könnte auf eines der Zugseile verzichtet werden. In dem Fall könnte dann eine Auslenkung in die eine Richtung durch Druck und die Auslenkung in die entgegengesetzte Richtung durch Zug des Zug-Druckelementes erfolgen.

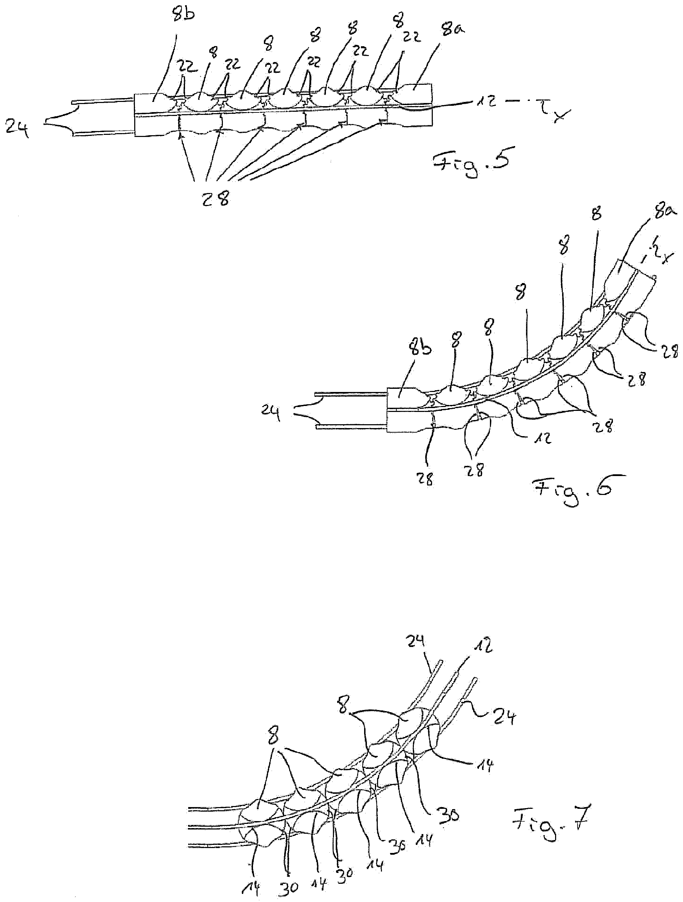

Auch ist eine Ausführungsform denkbar, bei welcher eine Auslenkung nur in eine Richtung möglich ist. Eine solche Ausführungsform ist in Fig. 5 gezeigt. Dort erstrecken sich die Verzahnungen 22 im Wesentlichen nur in 90°-Bögen und nicht halbkreisförmig, wie in den vorangehenden Figuren gezeigt. Die Verzahnungen erstrecken sich somit nur an einer Seite der Spannseile 12. An der anderen Seite sind an den Schaftabschnitten 8 sich im Wesentlichen normal zur Längsachse X erstreckende Schultern 28 ausgebildet, welche bei der axialen Erstreckung, welche in Fig. 5 gezeigt ist, bereits aneinander anliegen. D. h. bei dem Ausführungsbeispiel gemäß Fig. 5 kann eine Auslenkung nur in Fig. 5 nach oben hin erfolgen durch Kürzung des oberen Zugseils 24, während das untere Zugseil 24 in Fig. 5 gelängt wird. Eine Streckung in die gerade Richtung erfolgt dann durch Zug an dem unteren Zugseil 24. Der entsprechend gekrümmte bzw. ausgelenkte Zustand ist in Fig. 6 gezeigt. Bei der Krümmung bilden sich dann zwischen den Schultern 28 Spalte.

Ein weiteres Ausführungsbeispiel, bei welchem die Schaftabschnitte 8 keine Verzahnungen aufweisen sondern kraftschlüssig über Reibflächen 30 aneinander anliegen, zeigt Fig. 7. Im Übrigen entspricht der Aufbau dem vorangehend beschriebenen, d. h. auch hier werden die Schaftabschnitte 8 durch Spannstangen bzw. Spannseile 12 in Anlage gehalten. Auch hier rollen bei der Auslenkung bzw. Ausbiegung die benachbarten Schaftabschnitte 8 aufeinander ab, wie es vorangehend anhand der Verzahnungen 22 beschrieben wurde. Die Reibflächen 30 sind dabei entsprechend der vorangehend beschrieben Verzahnungen 22 kreisbogenförmig gekrümmt und von ihrer Oberflächenbeschaffenheit so ausgebildet, dass ein Schlupf bzw. ein Rutschen zwischen den Schaftabschnitten 8 verhindert wird. So wird auch bei diesem Ausführungsbeispiel wie bei den vorangehend beschrieben Ausführungsbeispielen mit den Verzahnungen eine geometrisch definierte Auslenkung des Schaftes erzielt.

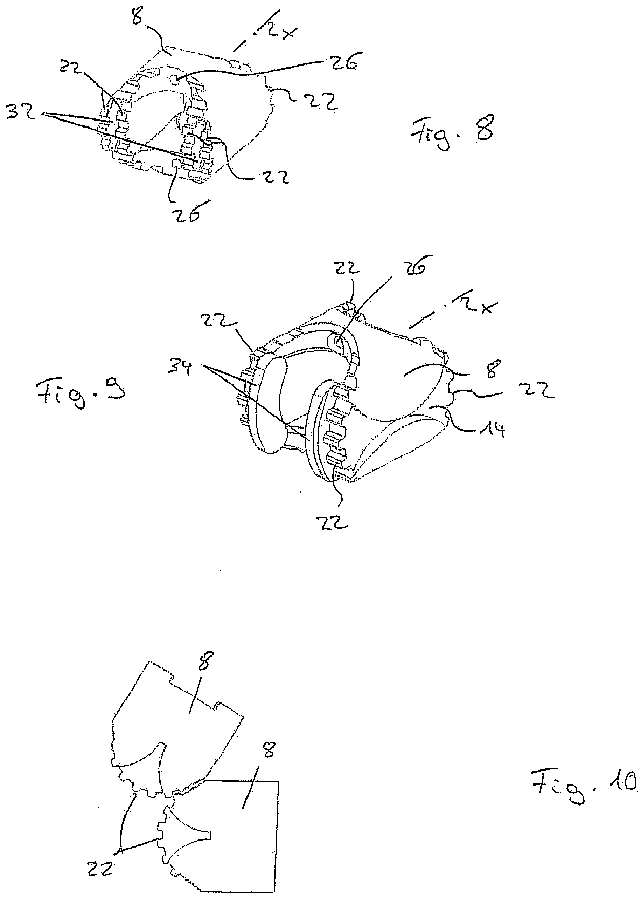

Fig. 8 zeigt am Beispiel eines einzelnen Schaftabschnittes 8 eine andere Art der Führung für die Spannseile 12. Bei dieser Ausführungsform sind keine Führungsnuten 14 vorgesehen sondern im Inneren der Seitenwandungen Führungskanäle 32 vorgesehen, welche sich in axialer Richtung X durch die Wandungen erstrecken. Hier erstrecken sich die Führungskanäle 32 durch die Umfangswandabschnitte, an deren Axialenden die Verzahnungen 22 ausgebildet sind. D. h. hier ist im Mittelbereich der Verzahnungen 22 eine Öffnung zu den Führungskanälen 32 gebildet. Erkennbar sind in Fig. 8 auch die Löcher 26, durch welche die Zugseile 24 geführt werden.

Fig. 9 zeigt eine weitere Variante des Aufbaus eines einzelnen Schaftabschnittes 8, bei welchem am Innenumfang seitlich der Verzahnungen 22 Führungsflächen 34 ausgebildet sind, welche in axialer Richtung X über die axialen Stirnkanten der Verzahnungen 22 vorstehen. Die Führungsflächen 34 sind nur an einem Axialende der Schaftabschnitte 8 ausgebildet, sodass sie bei einem benachbarten Schaftabschnitt 8 von dem entgegengesetzten Axialende her, an welchem keine Führungsflächen 34 ausgebildet sind, in das Innere des Schaftabschnittes 8 eingreifen können und am Innenumfang seitlich der Verzahnungen 22 zur Anlage kommen. Auf diese Weise bilden die Führungsflächen 34 eine seitliche Führung für die benachbarten Schaftabschnitte 8, sodass diese sich in einer Richtung der Krümmungsachsen der Verzahnungen 22 quer zur Längsachse X nicht gegeneinander bewegen können.

Fig. 10 zeigt eine Minimalkonfiguration eines abwinkelbaren Schaftbereiches, welche aus zwei aneinander angrenzenden zueinander verschwenkbaren Schaftabschnitten 8 gebildet wird. Diese Schaftabschnitte 8 sind über Verzahnungen 22 miteinander in Eingriff. Es ist zu erkennen, dass bei dieser Ausgestaltung, welche ein Abrollen der Schaftabschnitte 8 über ihre Verzahnungen 22 ermöglicht, eine Abwinklung von 120° erreicht werden kann. Die Anordnung von mehr Schaftabschnitten 8, wie beispielsweise in Fig. 4 gezeigt ist, ermöglicht einen ähnlichen Winkel bei größerem Radius.

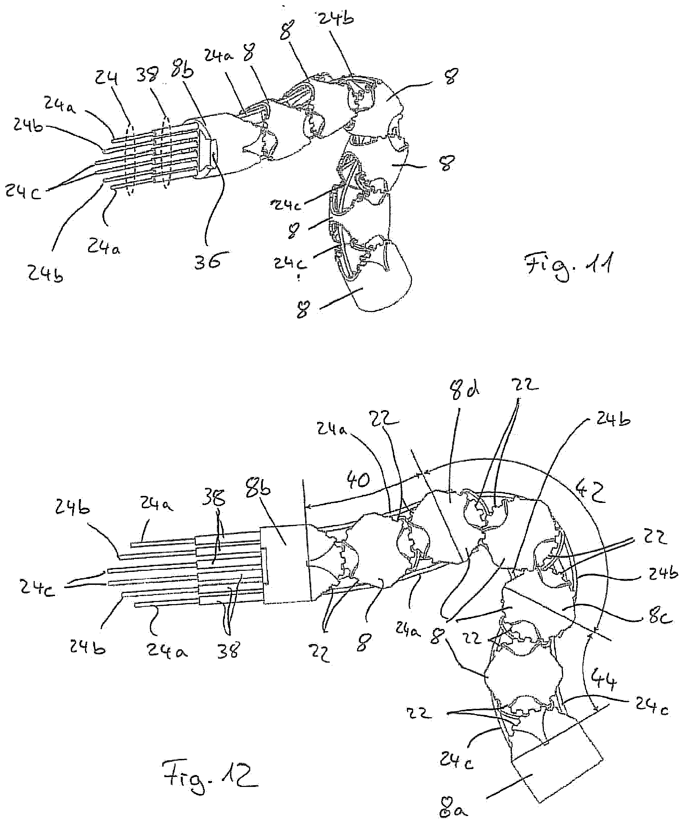

Die Fig. 11 und 12 zeigen eine weitere bevorzugte Ausführungsform, bei welcher der Schaftbereich sequentiell abwinkelbar ist. Auch hier ist der bewegliche bzw. abwinkelbare Schaftbereich 4 aus mehreren axial aneinander angrenzenden Schaftabschnitten 8 gebildet, welche durch hier nicht gezeigte Spannseile zusammengehalten werden, welche durch axiale Löcher 36 in den Wandungen der Schaftabschnitte 8 geführt sind. Die Löcher 36 erstrecken sich axial durch die Wandungen der Schaftabschnitte 8 an zwei diametral entgegengesetzten Seiten hindurch, ähnlich den Führungskanälen 32 in Fig. 8. Von den vorangehend beschriebenen Ausführungsbeispielen unterscheidet sich die Ausführungsform gemäß Fig. 11 und 12 darin, dass hier drei Paare von Zugseilen 24a, 24b und 24c vorhanden sind. Die Zugseile 24 sind jeweils in einer Hülle 38 nach Art eines Bowdenzuges geführt. Die Hülle dient der Abstützung der Zugseile 24 am proximal ersten Schaftabschnitt 8b. Dazu sind die Hüllen 38 in den Durchführungen im proximalen Schaftabschnitt 8d fixiert.

Durch die drei Paare von Zugseilen 24a, 24b und 24c können in dem Schaftbereich 4 drei Abwinkelbereiche 40, 42 und 44 realisiert werden. Die Zugseile 24c sind an zwei diametral entgegengesetzten Seiten des distalen Schaftabschnittes 8a festgelegt, wie die Zugseile 24 gemäß der vorangehenden Ausführungsbeispiele. Gleichzeitig sind die Hüllen 38 der Zugseile 24c in dem Schaftabschnitt 8c, welcher den Übergang zwischen den Abwinkelbereichen 42 und 44 bildet, fixiert. Die Zugseile 24b sind mit ihren distalen Enden in dem Schaftabschnitt 8c fixiert, während sich die Hüllen 38 dieser beiden Zugseile 24c an dem Schaftabschnitt 8d abstützen, welcher den Übergang zwischen den Abwinkelbereichen 40 und 42 bildet. Die Zugseile 24a sind mit ihren distalen Enden an dem Schaftabschnitt 8d befestigt und die Hüllen 38 der Zugseile 24a sind in dem Schaftabschnitt 8d festgelegt. Durch diese Ausgestaltung können die Abwinkelbereiche 40, 42 und 44 individuell ausgelenkt werden. Um die in den Fig. 11 und 12 gezeigte Auslenkung zu erreichen, wird in Fig. 12 das obere Zugseil 24a gekürzt und gleichzeitig das untere Zugseil 24a gelängt. Dadurch wird der erste Abwinkelbereich 40 zwischen den Schaftabschnitten 8b und 8d nach oben gekrümmt. Dabei rollen die Schaftabschnitte 8 und ihre Verzahnungen 22, wie oben beschrieben, aufeinander ab. Gleichzeitig ist das untere der Zugseile 24b in Fig. 12 gekürzt und das obere Zugseil 24b gelängt, sodass der zweite Abwinkelbereich 42 nach unten gekrümmt wird, wobei die Schaftabschnitte 8c und 8d sowie der weitere Schaftabschnitt 8, welcher zwischen diesen gelegen ist, über ihre Verzahnungen 22 aufeinander abrollen. Zur Krümmung des dritten Abwinkelbereiches 44 ist in Fig. 12 das obere Zugseil 24c wiederum gekürzt und das untere Zugseil 24c gelängt, sodass der dritte Abwinkelbereich 44 in Fig. 12 nach rechts bzw. nach oben gekrümmt ist. Dabei rollen die Schaftabschnitte 8c und 8a mit ihren Verzahnungen 22 auf dem zwischenliegenden Schaftabschnitt 8 ab. Es ist zu verstehen, dass durch Anordnung von entsprechend mehr Abschnitten 8 und einzelnen Zugseilen 24 auch mehr als drei individuell abwinkelbare Schaftbereiche geschaffen werden können. Auch ist es denkbar, lediglich zwei Abwinkelbereiche vorzusehen.

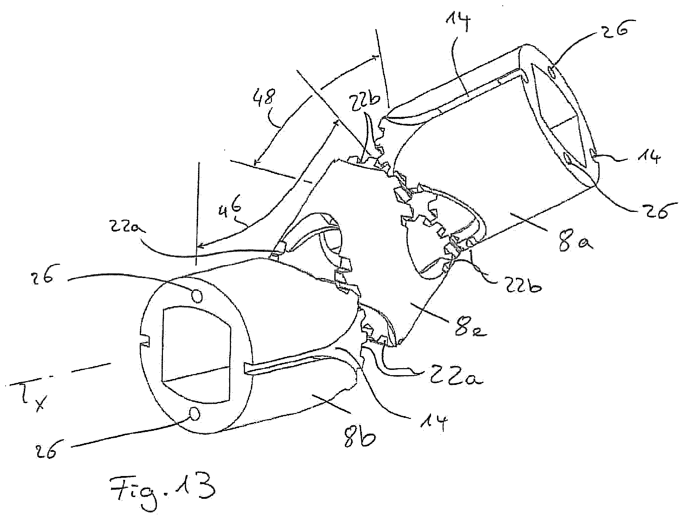

Fig. 13 zeigt nun eine Ausführungsform, bei welcher der Schaftbereich 4 in zwei unterschiedlichen Richtungen abwinkelbar ist. Bei diesem Ausführungsbeispiel sind lediglich drei Schaftabschnitte 8a und 8b sowie ein zwischenliegender Schaftabschnitt 8e gezeigt. Bei dieser Ausführungsform sind die Verzahnungen 22a zwischen den Schaftabschnitten 8b und 8e in einer ersten Richtung gekrümmt, d. h. um Achsen gekrümmt, welche in einer ersten Durchmesserrichtung bezogen auf die Längsachse X des Schaftes gerichtet sind. Die Verzahnungen 22b zwischen den Schaftabschnitten 8a und 8e, welche ebenfalls bogenförmig sind, sind in einer zweiten Richtung gekrümmt, d. h. jeweils um eine Achse gekrümmt, welche sich normal zu den Krümmungsachsen der Verzahnungen 22a in diametraler Richtung zur Längsachse X des Schaftes erstreckt. So sind die Abschnitte 8e und 8b in einer ersten Schwenkebene zueinander beweglich, während die Schaftabschnitte 8a und 8e in einer zweiten, sich rechtwinklig zu der ersten Schwenkebene 46 erstreckenden Schwenkebene 48 zueinander beweglich sind. Die Schwenkbewegungen werden durch Zugseile 24 veranlasst, welche in der anhand von Fig. 11 und 12 beschriebenen Weise sequenziell an einzelnen Schaftabschnitten 8 befestigt sein können oder aber auch alle am distalen Schaftabschnitt 8a befestigt sein können, sodass der Schaft zwar in zwei Richtungen aber lediglich in einem Abwinkelbereich abgewinkelt werden kann. Die Zugseile 24 und die Spannseile 12 erstrecken sich durch die Führungen 14 und die Löcher 26 sowie geeignete, hier nicht gezeigte Löcher bzw. Führungskanäle in dem Schaftabschnitt 8e. Es ist darüber hinaus zu verstehen, dass auch der Abwinkelbereich bei der Ausführungsform gemäß Fig. 13 länger ausgebildet werden kann, indem mehrere Schaftelemente 8e mit zueinander verdrehten Verzahnungen 22a und 22b zwischen dem distalen Schaftabschnitt 8a und dem proximalen Schaftabschnitt 8b angeordnet werden können. Dabei werden die einzelnen Schaftabschnitte 8e, welche aneinander angrenzen, jeweils um 90° um die Längsachse X zueinander verdreht angeordnet.

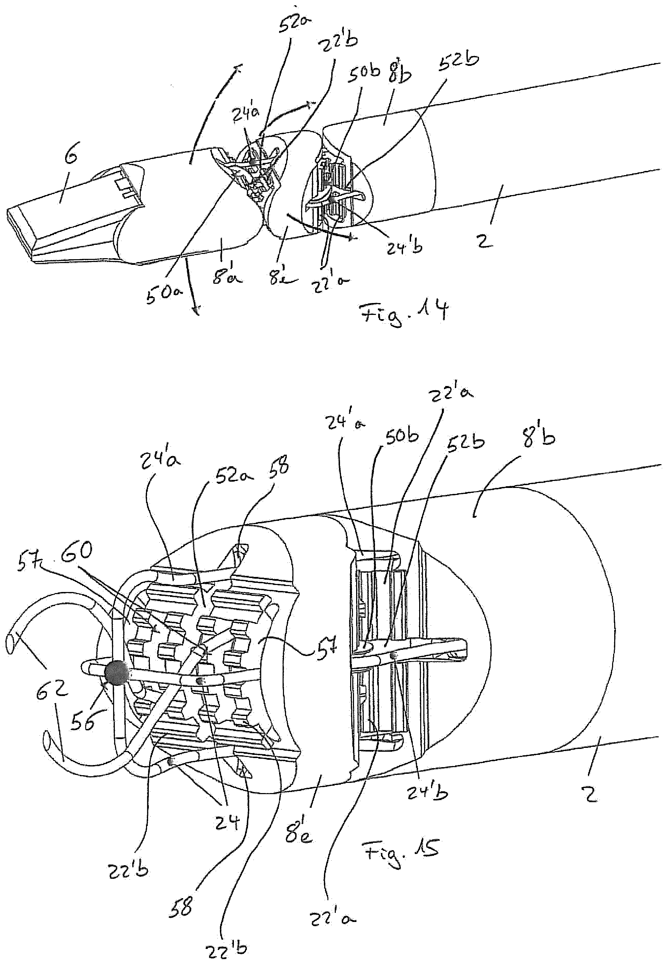

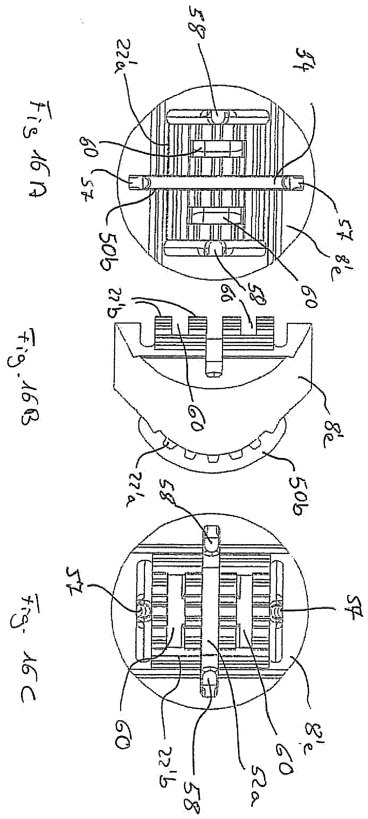

Anhand der Figuren 14 - 16 wird eine weitere Ausführungsform der Erfindung beschrieben. Der in Fig. 14 gezeigte Schaft 2 weist an seinem distalen Ende ein Zangenmaul 6 auf. Dabei ist das Zangenmaul 6 an einem distal beweglichen Schaftabschnitt 8'a angeordnet. Dieser Schaftabschnitt 8'a ist über einen zwischengelagerten weiteren beweglichen Schaftabschnitt 8'e mit einem Schaftabschnitt 8'd verbunden, welcher fest an dem starren Teil des Schaftes 2 angeordnet ist. Die Schaftabschnitte 8'b und 8'e rollen über Verzahnungen 22'a aufeinander ab. Die Schaftabschnitte 8'e und 8'a rollen über Verzahnungen 22'b aufeinander ab. Dabei sind die Verzahnungen 22'a und 22'b in zwei um 90° um die Längsachse des Schaftes 2 zueinander gedrehten Ebenen angeordnet, sodass der Schaftabschnitt 8'a gegenüber dem Schaftabschnitt 8'e in einer Ebene abgewinkelt wird, welche um 90° versetzt zu der Ebene ist, in welcher der Schaftabschnitt 8'e bzgl. dem Schaftabschnitt 8'b abgewinkelt wird. Die Verzahnungen 22'b und 22'a erstrecken sich hier im Wesentlichen über den gesamte Zentralbereich der Schaftabschnitte 8' und sind lediglich von Öffnungen durchbrochen.

Insbesondere ist bei dieser Ausführungsform eine zentrale Seitenführung vorgesehen, welche durch vorstehende Stege 50a und 50b gebildet werden. Der Steg 50a erstreckt sich bogenförmig am proximalen Ende des Schaftabschnittes 8'a parallel zu der Schwenkebene zwischen dem Schaftabschnitt 8'a und dem Schaftabschnitt 8'e. Der Steg 50b erstreckt sich ausgehend von der proximalen Seite des Schaftabschnittes 8'e, dem Schaftabschnitt 8'b zugewandt, parallel zu der Schwenkebene zwischen den Schaftabschnitten 8'e und 8'b. Der Steg 50a greift in eine Ausnehmung bzw. Nut 52a ein, welche an der dem Schaftabschnitt 8'a zugewandten Distalseite des Schaftabschnittes 8'e ausgebildet ist. Entsprechend greift der Steg 50b in eine Nut 52b ein, welche an der dem Schaftabschnitt 8'e zugewandten Distalseite des Schaftabschnittes 8'b ausgebildet ist. Die Nut 52a erstreckt sich dabei parallel zu dem Steg 50a, die Nut 52b erstreckt sich parallel zu dem Steg 50b. Durch den Eingriff der Stege 50 in die zugehörigen Nuten 52 wird in dem jeweiligen Gelenk eine Seitenführung quer zu der jeweiligen Schwenkebene realisiert.

Die Vorsprünge bzw. Stege 50a und 50b dienen gleichzeitig der Führung der Zugseile 24'a und 24'b. Dazu sind die Stege 50a und 50b auf ihren Außen- bzw. Oberflächen mit rinnenförmigen Vertiefungen 54 versehen, welche Seilführungen bilden. Hier sind zwei Paare von Zugseilen 24'a und 24'b vorgesehen, wobei das Paar von Zugseilen 24'b zur Auslenkung des Schaftabschnittes 8'e gegenüber dem Schaftabschnitt 8'b und das Paar von Zugseilen 24'a zur Auslenkung des Schaftabschnittes 8'a gegenüber dem Schaftabschnitt 8'e dienen. Die beiden Paare von Zugseilen 24' sind in dem distalen Schaftabschnitt 8'a an dem Verbindungspunkt 56 fest miteinander verbunden. Dies erleichtert die Montage, da die Zugseile 24' vom distalen Ende durch sämtliche Schaftabschnitte 8 gefädelt werden können. Die Zugseile 24'b erstrecken sich durch Löcher 57 seitlich des Steges 50b, während sich die Zugseile 24'a durch die Löcher 58 an den Längsenden der Nut 52a erstrecken. Die Löcher 57 und 58 erstrecken sich in axialer Richtung durch den Schaftabschnitt 8'e hindurch, wobei die Löcher 58' sich zum proximalen Ende hin und die Löcher 57 zum distalen Ende hin erweitern. Darüber hinaus sind weiter innenliegend Löcher 60 in dem mittleren Schaftabschnitt 8'e ausgebildet, durch welche sich Zugseile- bzw. Steuerseile 62 erstrecken, welche Betätigungsseile zum Öffnen und Schließen des Zangenmaules 6 bilden. Die Löcher 60 bilden Führungen für die Zugseile 62, sodass sich die Zugseile 62 im Inneren des Schaftes im Zentralbereich durch diesen in axialer Richtung hindurch erstrecken.

Hinsichtlich der vorangehend beschriebenen Ausführungsformen ist zu verstehen, dass alternativ bei allen gezeigten Ausführungsbeispielen eine Ausgestaltung mit Reibflächen, wie sie in Fig. 7 gezeigt ist, Verwendung finden könnte.

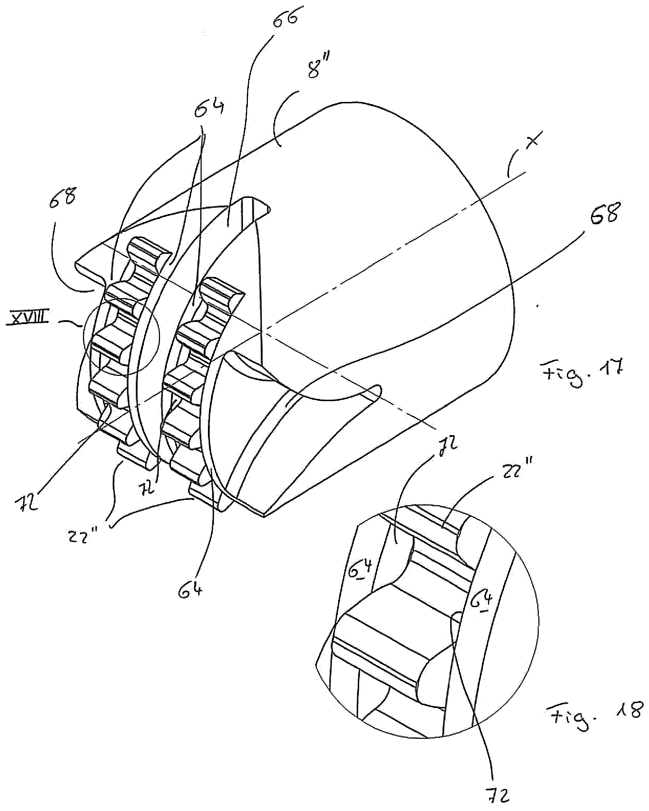

Bei der in Fig. 17 bis 20 sowie den Fig. 21 bis 25 gezeigten Ausführungsformen ist neben einer Verzahnung ein Kontakt über Anlageflächen zweier benachbarter Schaftabschnitte gegeben. Bei der Ausführungsform gemäß Fig. 17 bis 20 sind an dem Ende des Schaftabschnittes 8" zwei bogenförmige Verzahnungen 22" angeordnet. Parallel zu diesen bogenförmigen Verzahnungen 22" erstrecken sich an beiden Seiten jeder Verzahnung 22" bogenförmige Anlageflächen 64. Die Anlageflächen 64 erstrecken sich in radialer Richtung zwischen Kopf- und Fußkreis der Verzahnungen 22", bevorzugt auf dem Teilkreis. So stehen die Köpfe der Zähne der Verzahnungen 22" radial über die Anlagefläche 64 nach außen vor, sodass die Seitenflächen der Zähne mit den Seitenflanken 72 an der gegenüberliegenden Verzahnung eines angrenzenden Schaftabschnittes zur Begrenzung der seitlichen Bewegung in Anlage treten können. Zwischen den inneren Anlageflächen 64 zwischen den beiden Verzahnungen 22" erstreckt sich eine Nut bzw. ein Schlitz 66, durch welchen sich, wie oben anhand der Nut 52a und der Löcher 58 beschrieben, Zugseile erstrecken können. Entsprechend sind an den Außenseiten der äußeren Anlageflächen 64 seitlich der Verzahnungen 22 Nuten bzw. Ausnehmungen 68 vorgesehen, durch welche sich Spannseile oder Zugseile, wie sie oben als Spannseile 12 und Zugseile 24 sowie 24' beschrieben wurden, erstrecken können.

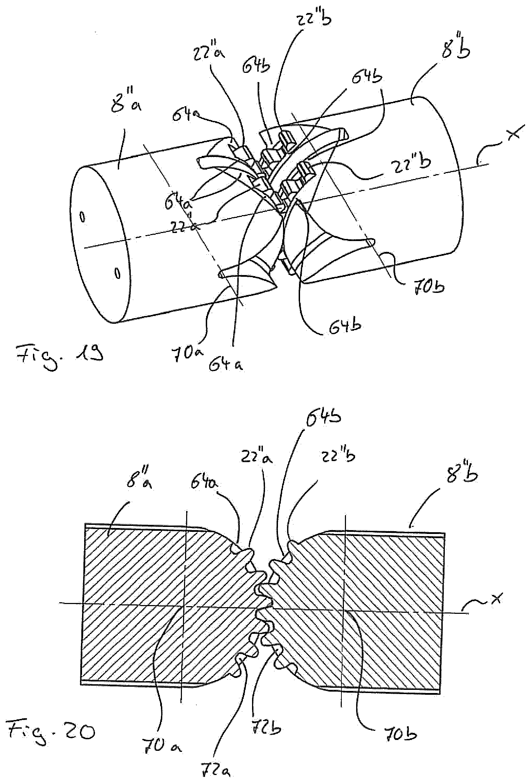

Wenn, wie in Fig. 19 gezeigt, zwei der Schaftabschnitte, wie sie in Fig. 17 und 18 gezeigt sind, einander gegenüberliegend angeordnet sind, greifen die Verzahnungen 22"a und 22"b, welche einander gegenüberliegen, wie oben anhand der anderen Ausführungsformen beschrieben, kämmend ineinander ein. Gleichzeitig liegen die einander gegenüberliegenden Anlageflächen 64a und 64b aneinander an. So rollen bei einer Abwinkelung die Anlageflächen 64a und 64b aufeinander ab. Gleichzeitig rollen die Verzahnungen 22"a und 22"b kämmend ineinander ab. Durch die Verzahnungen 22" wird dabei ein Formschluss erreicht, welcher ein definiertes Abrollen ohne Schlupf gewährleistet. Die Anlageflächen 64 halten dabei die ineinander kämmenden Verzahnungen 22"a und 22"b in definiertem Abstand zueinander, das heißt, die Dreh- bzw. Schwenkachsen 70a und 70b, welche den Mittelpunkt des Bogens, entlang dem sich die Verzahnungen 22"a und 22"b erstrecken, bilden, werden in definiertem Abstand zueinander gehalten.

Die Seitenflanken 72 (72a, 72b), welche sich normal zu den Anlageflächen 64 erstrecken und den Verzahnungen 22" zugewandt sind, bilden eine seitliche Führung, welche ein Verschieben der beiden Schaftabschnitte 8"a und 8"b entlang der Schwenkachsen 70a und 70b verhindern. Die Seitenflächen der Zähne der Verzahnungen 22"a und 22"b stoßen an diese inneren Seitenflanken 72 der Anlageflächen 64 an und bilden somit ein die seitliche Bewegung begrenzenden Anschlag.

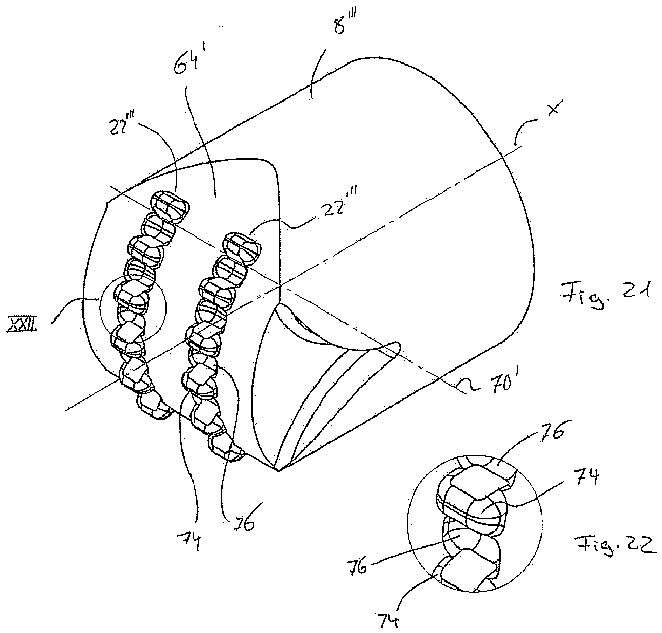

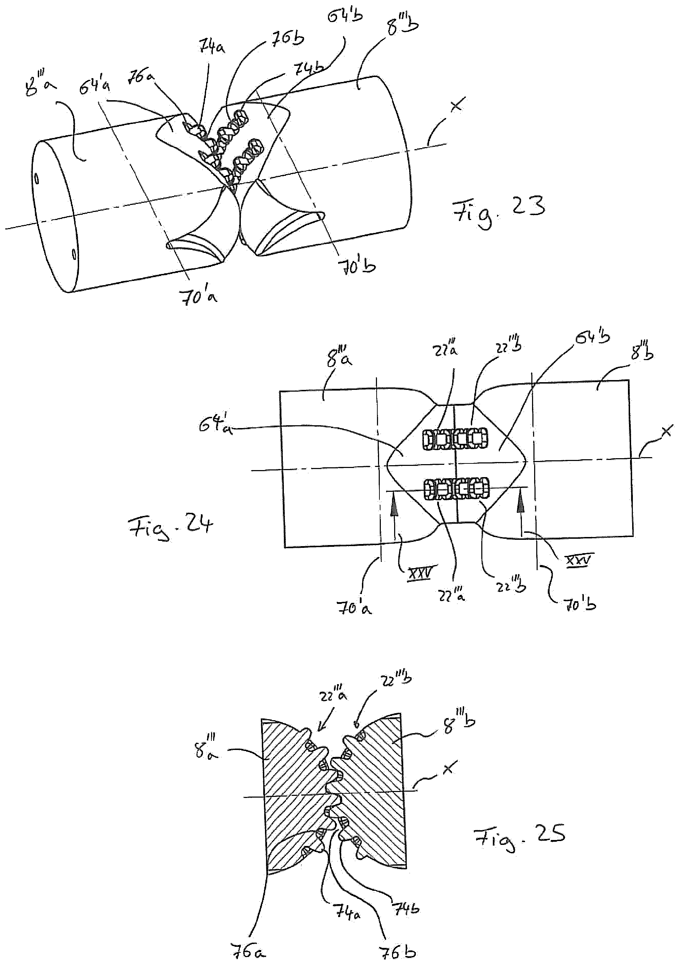

Die Fig. 21 bis 25 zeigen eine weitere Ausführungsform der Erfindung ähnlich der vorangehend beschriebenen Ausführungsform. Bei dieser Ausführungsform weist der Endbereich des Schaftabschnittes 8''' eine bogenförmige Anlagefläche 64' auf, welche um die Schwenkachse 70' kreisbogenförmig gekrümmt ist. In dieser Anlagefläche 64' sind zwei zueinander parallele bogenförmige Verzahnungen 22''' ausgebildet. Die Verzahnungen sind durch sich abwechselnde Zähne 74 und Mulden 76 gebildet. Die Form der Mulden 76 ist dabei an die Form der Zähne 74 angepasst, sodass die Zähne 74a eines ersten Schaftabschnittes 8'''a in die gegenüberliegenden Mulden 76b eines angrenzenden bzw. gegenüberliegenden zweiten Schaftabschnittes 8'''b eingreifen können und umgekehrt die Zähne 74b des zweiten Schaftabschnittes 8'''b in die Mulden 76a des ersten Schaftabschnittes 8'''a eingreifen, wie in den Fig. 23 bis 25 gezeigt ist. Auch bei dieser Ausführungsform rollen die beiden Schaftabschnitte 8'''a und 8'''b über ihre Anlageflächen 64'a und 64'b aufeinander ab, während die Verzahnungen 22'''a und 22"'b, welche in der vorangehend beschriebenen Weise durch Zähne 74 und Mulden 76 gebildet werden, kämmend ineinander greifen. So wird durch die Verzahnungen 22''' ein formschlüssiger, schlupfverhindernder Eingriff realisiert, während der Kontakt der Anlageflächen 64' die Schwenkachsen 70a' und 70b' der Schaftabschnitte 8'''a und 8'''b in definiertem Abstand zueinander hält, sodass die Verzahnungen 22'''a und 22'''b in gewünschter Weise ohne zu blockieren ineinander kämmen können.

Die Zähne 74 und Mulden 76 sind so ausgebildet, dass ihre in Richtung der Schwenkachsen 70' gelegenen Seiten gerundet ausgebildet sind. Dadurch wird eine gewisse Zentrierung in Richtung der Schwenkachsen 70' beim Eingriff der Zähne 74 in die Mulden 76 erreicht. Auch bei dieser Ausführungsform erstreckt sich die bogenförmige Anlagefläche 64' vorzugsweise auf dem Teilkreis der so gebildeten Verzahnungen 22'''.

Es ist zu verstehen, dass auch bei den Ausführungsformen gemäß Fig. 17 bis 25 die Führung der Zug- und Spannseile in einer in den vorangehenden Ausführungsformen beschriebenen Weise erfolgen kann. Auch die Anordnung mehrerer Schaftabschnitte kann in der Weise erfolgen, wie es oben beschrieben wurde. Auch sind mit diesen Ausführungsformen sich abwechselnde Schwenkrichtungen, wie sie anhand der Fig. 13 bis 16 beschrieben wurden, möglich, indem die Schwenkachsen bzw. Krümmungsachsen, um welche die Anlageflächen 64, 64' und Verzahnungen 22" und 22''' verschwenken, zwischen einzelnen Schaftabschnitten jeweils in einem bestimmten Winkel, vorzugsweise 90° versetzt zueinander angeordnet werden. Insofern wird auf die obige Beschreibung verwiesen.

**Bezugszeichenliste**

| | | |
|---|---|---|
| 2 | - | Schaft |
| 4 | - | distaler Schaftbereich |
| 6 | - | Zangenmaul |
| 8, 8a, 8b, 8c, 8d, 8e, 8', 8'a, 8'b, 8'e, 8", 8"a, 8"b, 8''', 8'''a, 8'''b | - | Schaftabschnitte |
| 10 | - | Schutzschlauch |
| 12 | - | Spannseile |
| 14 | - | Führungsnute |
| 16 | - | Druckfedern |
| 18 | - | Hülsen |
| 20 | - | proximale Endfläche |
| 22, 22a, 22b, 22', 22'a, 22'b, 22", 22"a, 22"b, 22''', 22'''a, 22'''b | - | Verzahnungen |
| 24, 24a, 24b,24c, 24', 24'a, 24'b | - | Zugseile |
| 26 | - | Löcher |
| 28 | - | Schultern |
| 30 | - | Reibflächen |
| 32 | - | Führungskanäle |
| 34 | - | Führungsflächen |
| 36 | - | Löcher |
| 38 | - | Hüllen |
| 40, 42, 44 | - | Abwinkelbereiche |
| 46 | - | erste Schwenkebene |
| 48 | - | zweite Schwenkebene |
| 50a, 50b | - | Steg |
| 52a, 52b | - | Nuten |
| 54 | - | Vertiefungen |
| 56 | - | Verbindungspunkt |
| 57, 58, 60 | - | Löcher |
| 62 | - | Zugseile |
| 64, 64a, 64b, 64', 64'a, 64'b | - | Anlageflächen |
| 66 | - | Schlitz |
| 68 | - | Ausnehmungen |
| 70, 70a, 70b, 70', 70'a, 70'b | - | Schwenkachsen, |
| 72, 72a, 72b | - | Seitenflanken |
| 74, 74a, 74b | - | Zähne |
| 76, 76a, 76b | - | Mulden |
| | | |
| X | - | Längsachse des Schaftes |

## Patentansprüche

1. Medizinisches Instrument mit einem Schaft (2), welcher zumindest zwei benachbarte, gegeneinander bewegliche Schaftabschnitte (8) aufweist, wobei die zumindest zwei beweglichen Schaftabschnitte (8) reibschlüssig aneinander abrollend oder über Verzahnungen (22; 22'; 22"; 22''') kämmend miteinander in Eingriff sind, wobei die Verzahnungen (22; 22'; 22"; 22''') oder Reibflächen (30) bogenförmig um Achsen gekrümmt ausgebildet sind, wobei die miteinander kämmenden Verzahnungen (22; 22'; 22"; 22''') oder Reibflächen (30) an den einander gegenüberliegenden Stirnenden zweier benachbarter Schaftabschnitte (8; 8'; 8"; 8''') in entgegengesetzten Richtungen gekrümmt sind,
wobei sich zumindest zwei in Axialrichtung des Schaftes (2) erstreckende Zugseile (24) an zwei diametral entgegengesetzten Seiten vorhanden sind,
wobei eine diametrale Verbindungslinie zwischen den Zugseilen (24) sich normal zu den Achsen erstreckt, um welche sich die Verzahnungen (22) oder Reibflächen (30) krümmen,
**dadurch gekennzeichnet, dass** sich in Axialrichtung des Schaftes (2) erstreckende und über Federelemente vorgespannte Spannseile (12) an zwei diametral entgegengesetzten Seiten vorhanden sind, wobei die Spannseile (12) die einzelnen Schaftabschnitte mit ihren Verzahnungen oder Reibflächen in Anlage bzw. in Eingriff halten, wobei die Spannseile (12) an einem distalen Schaftabschnitt (8a) fixiert sind und am entgegengesetzten Axialende auf den Spannseilen (12) Druckfedern (16) angeordnet sind, welche sich mit einem Axialende an einer Hülse (18), welche auf dem Spannseil (12) fixiert ist, abstützen und mit dem entgegengesetzten Axialende an einer proximalen Endfläche (20) eines proximalen Schaftabschnittes (8b) abstützen,
wobei die Zugseile (24) an zwei diametral entgegengesetzten Seiten vorhanden sind, welche um 90° versetzt zu den Seiten angeordnet sind, an welchen die Spannseile (12) angeordnet sind.

2. Medizinisches Instrument nach Anspruch 1, **dadurch gekennzeichnet, dass** der Schaft (2) eine Mehrzahl gegeneinander beweglicher Schaftabschnitte (8) aufweist, von denen jeweils zwei zueinander benachbarte Schaftabschnitte (8) über Verzahnungen (22; 22'; 22"; 22''') oder Reibflächen (30) kämmend miteinander in Eingriff sind.

3. Medizinisches Instrument nach Anspruch 1 oder 2, **dadurch gekennzeichnet, dass** zwei benachbarte Schaftabschnitte (8) an ihren einander gegenüberliegenden Stirnenden mit korrespondierenden Verzahnungen (22; 22'; 22"; 22''') oder Reibflächen (30) versehen sind.

4. Medizinisches Instrument nach einem der vorangehenden Ansprüche, **dadurch gekennzeichnet, dass** die miteinander kämmenden Verzahnungen (22; 22'; 22"; 22''') oder Reibflächen (30) an den einander gegenüberliegenden Stirnenden zweier benachbarter Schaftabschnitte (8; 8'; 8"; 8''') denselben Krümmungsradius aufweisen.

5. Medizinisches Instrument nach einem der vorangehenden Ansprüche, **dadurch gekennzeichnet, dass** die benachbarten Schaftabschnitte (8; 8'; 8"; 8''') an ihren einander zugewandten Stirnenden jeweils an zwei diametral entgegengesetzten Seiten eine Verzahnung (22; 22'; 22"; 22''') oder Reibflächen (30) aufweisen.

6. Medizinisches Instrument nach Anspruch 5, **dadurch gekennzeichnet, dass** sich die Verzahnungen (22; 22'; 22"; 22''') oder Reibflächen (30) an den zwei diametral entgegengesetzten Seiten bogenförmig um dieselbe Achse erstrecken.

7. Medizinisches Instrument nach einem der vorangehenden Ansprüche, **dadurch gekennzeichnet, dass** der Schaft zumindest drei gegeneinander bewegliche Schaftabschnitte (8; 8'; 8"; 8''') aufweist, von denen die jeweils benachbarten Schaftabschnitte (8; 8'; 8"; 8''') über Verzahnungen (22; 22'; 22"; 22''') oder Reibflächen (30) kämmend miteinander in Eingriff sind, wobei der erste und der zweite Schaftabschnitt (8) in einer ersten Schwenkebene (46) und derzweite und der dritte Schaftabschnitt (8; 8'; 8"; 8''') in einer zweiten Schwenkebene (48) zueinander beweglich sind.

8. Medizinisches Instrument nach Anspruch 7, **dadurch gekennzeichnet, dass** die erste (46) und zweite (48) Schwenkebene sich normal zueinander erstrecken.

9. Medizinisches Instrument nach Anspruch 7 oder 8, **dadurch gekennzeichnet, dass** die Verzahnungen (22; 22'; 22"; 22''') oder Reibflächen (30) bogenförmig ausgebildet sind und die Verzahnungen (22; 22'; 22"; 22''') oder Reibflächen (30) zwischen dem ersten und dem zweiten Schaftabschnitt um Achsen gekrümmt sind, welche sich normal zu den Achsen erstecken, um welche die Verzahnungen (22; 22'; 22"; 22''') oder Reibflächen (30) zwischen dem zweiten und dem dritten Schaftabschnitt (8; 8'; 8"; 8''') gekrümmt sind.

10. Medizinisches Instrument nach einem der vorhergehenden Ansprüche, **dadurch gekennzeichnet, dass** die zumindest zwei beweglichen Schaftabschnitte (8"; 8''') über Verzahnungen (22"; 22''') kämmend miteinander in Eingriff sind und gleichzeitig über bogenförmige Anlageflächen (64; 64') aneinander abrollen.

11. Medizinisches Instrument nach Anspruch 10, **dadurch gekennzeichnet, dass** jeder Schaftabschnitt (8"; 8") zumindest eine bogenförmige Anlagefläche (64; 64') und eine Verzahnung (22"; 22''') aufweist, wobei die bogenförmige Anlagefläche (64, 64') parallel zu der Verzahnung (22"; 22''') gekrümmt ist.

12. Medizinisches Instrument nach Anspruch 11, **dadurch gekennzeichnet, dass** die bogenförmige Anlagefläche denselben Radius wie der Teilkreis der Verzahnung (22"; 22''') hat.

13. Medizinisches Instrument nach einem der vorangehenden Ansprüche, **dadurch gekennzeichnet, dass** die zumindest zwei Zugseile mit ihren distalen Enden an zwei diametral entgegengesetzten Seiten eines distalen Schaftabschnittes (8) befestigt sind.

14. Medizinisches Instrument nach Anspruch 13, **dadurch gekennzeichnet, dass** vier Zugseile (24) vorgesehen sind, welche um jeweils 90° bezüglich der Längsachse (X) versetzt an einem distalen Schaftabschnitt (8a) befestigt sind.

15. Medizinisches Instrument nach Anspruch 13 oder 14, **dadurch gekennzeichnet, dass** vier Zugseile (24) vorgesehen sind, welche an ihrem distalen Ende miteinander verbunden sind.

16. Medizinisches Instrument nach einem der Ansprüche 13 bis 15, **dadurch gekennzeichnet, dass** in denjenigen Schaftabschnitten (8a), welche proximalseitig des distalen Schaftabschnittes, an welchem die Zugseile (24) befestigt sind, gelegen sind, Führungen für die Zugseile (24) ausgebildet sind.

17. Medizinisches Instrument nach einem der vorangehenden Ansprüche, **dadurch gekennzeichnet, dass** die zumindest zwei benachbarten Schaftabschnitte (8) über zumindest eine sich parallel zur Schwenkebene erstreckende Seitenführung (50, 52) miteinander in Eingriff sind.

18. Medizinisches Instrument nach einem der vorangehenden Ansprüche, **dadurch gekennzeichnet, dass** die zumindest zwei beweglichen Schaftabschnitte jeweils einen zentralen, sich in axialer Richtung durch den Schaftabschnitt hindurch erstreckenden Hohlraum aufweisen, wobei die Hohlräume der zumindest zwei beweglichen Schaftabschnitte miteinander fluchten.

## Claims

1. A medical instrument with a shank (2) which comprises at least two adjacent shank sections (8) which are movable to one another, wherein the at least two movable shank sections (8) frictionally roll on one another or are meshingly engaged with one another via toothings (22, 22'; 22"; 22'''), wherein the toothings (22, 22'; 22"; 22''') or friction surfaces (30) are designed arcuately bent about axes, wherein the toothings (22, 22'; 22"; 22''') which mesh with one another or frictions surfaces (30) are bent in opposite directions at the face ends of two adjacent shank sections (8, 8'; 8"; 8''') which lie opposite one another, wherein at least two pull cables (24) extending in the longitudinal axis (X) of the shank (2) are present at two diametrically opposite sides, wherein a diametrical connection line between the pull cables (24) extends normally to the axes, about which the toothings (22) or friction surfaces (30) are bent, **characterised in that** tension cables (12) which extend in the axial direction of the shank (2) and are biased via spring elements are present on two diametrically opposite sides, wherein the tension cables (12) keep the individual shank sections with their toothings or friction surfaces in contact or in engagement, wherein the tensioning cables (12) are fixed on a distal shank section (8a) and compression springs (16) are arranged on the opposite axial end on the tension cables (12), said compression springs being supported with an axial end on a sleeve (18) which is fixed on the tension cable (12) and with the opposite axial end being supported on a proximal end face (20) of a proximal shank section (8b), wherein the pull cables (24) are present on two diametrically opposite sides which are arranged offset by 90° to the sides at which the tension cables (12) are arranged.

2. A medical instrument according to claim 1, **characterised in that** the shank (2) comprises a plurality of shank sections (8) which can be moved to one another and of which in each case two shank sections (8) which are adjacent to one another are meshingly engaged with one another via toothings (22, 22'; 22"; 22''') or friction surfaces (30).

3. A medical instrument according to claim 1 or 2, **characterised in that** two adjacent shank sections (8) at their face ends which lie opposite one another are provided with corresponding toothings (22, 22'; 22"; 22''') or friction surfaces (30).

4. A medical instrument according to one of the preceding claims, **characterised in that** the toothings (22, 22'; 22"; 22''') which mesh with one another or frictions surfaces (30) have the same radius of curvature at the face ends of two adjacent shank sections (8, 8'; 8"; 8''') which lie opposite one another.

5. A medical instrument according to one of the preceding claims, **characterised in that** the adjacent shank sections (8, 8'; 8"; 8''') at their face ends which face one another comprise a toothing (22, 22'; 22"; 22''') or friction surfaces (30), in each case on two diametrically opposite sides.

6. A medical instrument according to claim 5, **characterised in that** the toothings (22, 22'; 22"; 22''') or friction surfaces (30) extend in an arched manner about the same axis at the two diametrically opposite sides.

7. A medical instrument according to one of the preceding claims, **characterised in that** the shank comprises at least three shank sections (8, 8'; 8"; 8''') which can be moved to one another, of which the respective adjacent shank sections (8, 8'; 8"; 8''') are meshingly engaged with one another via toothings (22, 22'; 22"; 22''') or friction surfaces (30), wherein the first and the second shank section (8) are movable to one another in a first pivot plane (46) and the second and the third shank section (8, 8'; 8"; 8''') in a second pivot plane (48).

8. A medical instrument according to claim 7, **characterised in that** the first (46) and the second (48) pivot plane extend normally to one another.

9. A medical instrument according to claim 7 or 8, **characterised in that** the toothings (22, 22'; 22"; 22''') or friction surfaces (30) are designed in an arched manner, and the toothings (22, 22'; 22"; 22''') or friction surfaces (30) between the first and the second shank section are bent about axes which extend normally to the axes, about which the toothings (22, 22'; 22"; 22''') or friction surfaces (30) between the second and the third shank section (8, 8'; 8"; 8''') are bent.

10. medical instrument according to one of the preceding claims, **characterised in that** the at least two movable shank sections (8"; 8''') are meshingly engaged with one another via toothings (22"; 22''') and simultaneously roll on one another via arched contact surfaces (64; 64').

11. A medical instrument according to claim 10, **characterised in that** each shank section (8"; 8''') comprises at least one arched contact surface (64; 64') and a toothing (22"; 22'''), wherein the arched contact surface (64, 64') is bent parallel to the toothing (22", 22''').

12. A medical instrument according to claim 11, **characterised in that** the arched contact surface has the same radius as the part-circle of the toothing (22", 22''').

13. A medical instrument according to one of the preceding claims, **characterised in that** at least two pull cables (24) with their distal ends are fastened on two diametrically opposite sides of a distal shank section (8).

14. A medical instrument according to claim 13, **characterised in that** four pull cables (24) are provided, which are fastened on a distal shank section (8a) in a manner offset in each case by 90□ with respect to the longitudinal axis (X).

15. A medical instrument according to claim 13 or 14, **characterised in that** four pull cables (24) are provided, which are connected to one another at their distal end.

16. A medical instrument according to one of the claims 13 to 15, **characterised in that** guides for the pull cables (24) are formed in those shank sections (8a) which are situated proximally of the distal shank section, on which the pull cables (24) are fastened.

17. A medical instrument according to one of the preceding claims, **characterised in that** the at least two adjacent shank sections (8) are engaged with one another via at least one side guide (50, 52) which extends parallel to the pivot plane.

18. A medical instrument according to one of the preceding claims, **characterised in that** the at least two movable shank sections each comprise a central cavity which extends in the axial direction through the shank section, wherein the cavities of the at least two movable shank sections are aligned to one another.

## Revendications

1. Instrument médical comprenant une tige (2) qui comporte au moins deux sections de tige (8) avoisinantes mobiles l'une par rapport à l'autre, ou les unes par rapport aux autres, lesdites au moins deux sections de tige (8) mobiles étant en prise l'une avec l'autre, ou les unes avec les autres, par effet de friction et déroulant l'une sur l'autre, ou les unes sur les autres, ou par l'intermédiaire de dentelures (22 ; 22' ; 22" ; 22''') s'engrenant l'une dans l'autre, ou les unes dans les autres, les dentelures (22 ; 22' ; 22" ; 22''') ou surfaces de friction (30) étant formées de façon arquée autour d'axes, les dentelures (22 ; 22' ; 22" ; 22''') s'engrenant l'une dans l'autre, ou les unes dans les autres, ou surfaces de friction (30) étant courbées dans des directions opposées, aux faces frontales en regard l'une à l'autre de deux sections de tige (8; 8'; 8";8''') avoisinantes,
au moins deux câbles de traction (24), s'étendant dans la direction axiale de la tige (2), existant sur deux côtés diamétralement opposés,
une ligne de liaison diamétrale entre les câbles de traction (24) s'étendant perpendiculairement aux axes autour desquels les dentelures (22) ou surfaces de friction (30) sont courbées,
**caractérisé en ce que** des câbles de tension (12) s'étendant dans la direction axiale de la tige (2) et précontraints par l'intermédiaire d'éléments ressorts existent sur deux côtés diamétralement opposés, les câbles de tension (12) maintenant les différentes sections de tige avec leurs dentelures ou surfaces de friction en appui ou en prise, les câbles de tension (12) étant fixés à une section de tige distale (8a) et des ressorts de compression (16) étant disposés sur les câbles de tension (12) à l'extrémité axiale opposée, lesquels ressorts étant en appui, par une extrémité axiale, sur une douille (18) qui est fixée sur le câble de tension (12) et en appui, par l'extrémité opposée, sur une surface d'extrémité proximale (20) d'une section de tige proximale (8b), les câbles de traction (24) existant sur deux côtés diamétralement opposés qui sont décalés de 90° par rapport aux côtés sur lesquels les câbles de tension (12) sont disposés.

2. Instrument médical selon la revendication 1, **caractérisé en ce que** la tige (2) comporte une quantité de sections de tige (8) dont deux sections de tige (8) avoisinantes respectives sont en prise l'une avec l'autre en s'engrenant par l'intermédiaire de dentelures (22 ; 22' ; 22" ; 22''') ou de surfaces de friction (30).

3. Instrument médical selon la revendication 1 ou 2, **caractérisé en ce que** deux sections de tige avoisinantes (8) sont pourvues de dentelures (22, 22', 22", 22''') correspondantes ou de surfaces de friction (30) à leurs faces frontales en regard l'une à l'autre.

4. Instrument médical selon l'une des revendications précédentes, **caractérisé en ce que** les dentelures (22 ; 22' ; 22" ; 22''') s'engrenant l'une dans l'autre ou surfaces de friction (30) présentent le même rayon de courbure aux faces frontales en regard l'une à l'autre de deux sections de tige avoisinantes (8 ; 8' ; 8" ; 8''').

5. Instrument médical selon l'une des revendications précédentes, **caractérisé en ce que** les sections de tige avoisinantes (8 ; 8' ; 8" ; 8''') comportent une dentelure (22 ; 22' ; 22" ; 22''') ou des surfaces de friction à leurs faces frontales en regard l'une à l'autre sur deux côtés diamétralement opposés.

6. Instrument médical selon la revendication 5, **caractérisé en ce que** les dentelures (22 ; 22' ; 22" ; 22"') ou les surfaces de friction (30) s'étendent sur les deux côtés diamétralement opposés de façon arquée autour du même axe.

7. Instrument médical selon l'une des revendications précédentes, **caractérisé en ce que** la tige comprend au moins trois sections de tige mobiles les unes par rapport aux autres dont les sections de tiges avoisinantes (8 ; 8' ; 8" ; 8''') respectives sont en prise l'une avec l'autre en s'engrenant par l'intermédiaire de dentelures (22 ; 22' ; 22" ; 22''') ou de surfaces de friction (30), la première et la deuxième section de tige (8 ; 8' ; 8" ; 8''') étant mobiles l'une par rapport à l'autre dans un premier plan de basculement (46) et la deuxième et la troisième section de tige (8 ; 8' ; 8" ; 8''') dans un deuxième plan de basculement (48).

8. Instrument médical selon la revendication 7, **caractérisé en ce que** le premier (46) et le deuxième (48) niveau de basculement s'étendent perpendiculairement l'un par rapport à l'autre.

9. Instrument médical selon la revendication 7 ou 8, **caractérisé en ce que** les dentelures (22 ; 22' ; 22" ; 22''') ou surfaces de friction (30) sont formées de façon arquée et que les dentelures (22 ; 22' ; 22" ; 22''') ou surfaces de friction (30) sont courbées entre le première et la deuxième section de tige autour d'axes qui s'étendent perpendiculairement aux axes autour desquels les dentelures (22 ; 22' ; 22" ; 22''') ou surfaces de friction (30) sont courbées entre la deuxième et la troisième section de tige (8 ; 8' ; 8" ; 8''').

10. Instrument médical selon l'une des revendications précédentes, **caractérisé en ce que** lesdits au moins deux sections de tige mobiles (8" ; 8"') sont en prise l'une avec l'autre, ou les unes avec les autres, en s'engrenant par l'intermédiaire de dentelures (22" ; 22''') et, en même temps, déroulent l'une sur l'autre par l'intermédiaire de surfaces d'appui (64 ; 64') de forme arquée.

11. Instrument médical selon la revendication 10, **caractérisé en ce que** chaque section de tige (8" ; 8") comprend au moins une surface d'appui (64 ; 64') de forme arquée et une dentelure (22" ; 22'''), la surface d'appui (64 ; 64') de forme arquée étant courbée parallèlement à la dentelure (22" ; 22''').

12. Instrument médical selon la revendication 11, **caractérisé en ce que** la surface d'appui de forme arquée a le même rayon que le cercle gradué de la dentelure (22" ; 22''').

13. Instrument médical selon l'une des revendications précédentes, **caractérisé en ce que** lesdits au moins deux câbles de traction sont fixés par leur extrémités distales sur deux côtés diamétralement opposés d'une section de tige distale (8).

14. Instrument médical selon la revendication 13, **caractérisé en ce que** sont prévus quatre câbles de traction (24) qui sont fixés sur une section de tige distale (8a), chacun décalé de 90° par rapport à l'axe longitudinal (x).

15. Instrument médical selon la revendication 13 ou 14, **caractérisé en ce que** sont prévus quatre câbles de traction (24) qui sont reliés les uns aux autres à leur extrémité distale.

16. Instrument médical selon l'une des revendications 13 à 15, **caractérisé en ce que** dans les sections de tige (8a) qui sont situées du côté proximal de la section de tige distale à laquelle les câbles de traction (24) sont fixés, sont formés des guidages pour les câbles de traction (24).

17. Instrument médical selon l'une des revendications précédentes, **caractérisé en ce que** lesdits au moins deux sections de tige avoisinantes (8) sont en prise l'une avec l'autre, ou les unes avec les autres, par l'intermédiaire d'au moins un guidage latéral (50, 52) s'étendant parallèlement au plan de basculement.

18. Instrument médical selon l'une des revendications précédentes, **caractérisé en ce que** lesdites au moins deux sections de tige mobiles comprennent chacune une cavité centrale, s'étendant à travers la section de tige dans une direction axiale, les cavités desdites au moins deux sections de tige mobiles étant alignées entre elles.
